(19) Europäisches Patentamt
European Patent Office
Office européen des brevets

(11) **EP 0 681 468 B1**

## (12) EUROPÄISCHE PATENTSCHRIFT

(45) Veröffentlichungstag und Bekanntmachung des Hinweises auf die Patenterteilung:
**19.03.1997 Patentblatt 1997/12**

(21) Anmeldenummer: **94905094.2**

(22) Anmeldetag: **20.01.1994**

(51) Int. Cl.$^6$: **A61K 7/50**, A61K 7/48, A61K 7/06, C11D 1/83, C11D 3/37

(86) Internationale Anmeldenummer:
**PCT/EP94/00132**

(87) Internationale Veröffentlichungsnummer:
**WO 94/16678 (04.08.1994 Gazette 1994/18)**

(54) **OBERFLÄCHENAKTIVE MISCHUNGEN**

SURFACTANT MIXTURES

MELANGES TENSIOACTIFS

(84) Benannte Vertragsstaaten:
**AT BE CH DE DK ES FR GB GR IE IT LI NL PT SE**

(30) Priorität: **28.01.1993 DE 4302314**

(43) Veröffentlichungstag der Anmeldung:
**15.11.1995 Patentblatt 1995/46**

(73) Patentinhaber: **Henkel Kommanditgesellschaft auf Aktien**
**40191 Düsseldorf (DE)**

(72) Erfinder:
- **MÜLLER, Reinhard**
  **D-41812 Erkelenz (DE)**
- **SEIDEL, Kurt**
  **D-40589 Düsseldorf (DE)**
- **HOLLENBERG, Detlef**
  **D-40699 Erkrath (DE)**
- **EHLERT, Maunela**
  **D-51379 Leverkusen (DE)**

(56) Entgegenhaltungen:
EP-A- 0 384 983          EP-A- 0 511 466
EP-A- 0 555 155          WO-A-93/00417
GB-A- 2 255 101

EP 0 681 468 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 681 468 B1

**Beschreibung**

**Gebiet der Erfindung**

Die Erfindung betrifft oberflächenaktive Mischungen, insbesondere Mittel zum Waschen oder Spülen von Haaren, die aufgrund ihrer Zusammensetzung besonders hautschonend sind.

**Stand der Technik**

Zur Erzielung einer reinigenden Wirkung enthalten wäßrige Reinigungsmittel üblicherweise oberflächenaktive Verbindungen, wodurch in der Regel die Hautbelastung des Mittels erhöht wird. Dies gilt besonders für die wichtige Klasse der Aniontenside.

Diese erhöhte Hautbelastung sollte im Bereich der Körperreinigungsmittel vermieden werden. Insbesondere bei Produkten, die für eine häufige Anwendung konzipiert sind, zur Reinigung des Intimbereiches dienen oder mit Schleimhäuten in Berührung kommen, ist eine gute Hautverträglichkeit wichtig. Es besteht daher ein ständiger Bedarf an milden wäßrigen Reinigungsmitteln mit gutem Schaumvermögen.

Von besonderem Interesse auf dem Gebiet der wäßrigen Reinigungsmittel sind Mittel zum Waschen oder Spülen von Haaren, zum Beispiel Shampoos und abspülbare Haarnachbehandlungsmittel. Dabei besteht insbesondere ein ständiger Bedarf nach solchen Mitteln, die eine verbesserte Wirkung hinsichtlich Fülle und Frisierbarkeit der Haare aufweisen.

Das Haupthaar weist nach dem Waschen oft einen kosmetisch unbefriedigenden Zustand auf. Es fühlt sich stumpf an, ist im nassen Zustand nur schwer zu kämmen und neigt im trockenen Zustand zur statischen Aufladung wodurch das Kämmen erschwert und der Sitz des gekämmten Haares gestört wird.

Bekannt ist die Verwendung zwitterionischer Polymerer, die anionische Gruppen, in der Regel Carboxylgruppen, und quartäre Ammoniumgruppen im Molekül enthalten, in Haarbehandlungsmitteln. Beispielsweise beschreibt die DE-OS-21 50 557 die Verwendung von Polymerisaten zwitterionischer Monomerer in Haarfestlegemittel. Zwitterionische Polymere zeigen jedoch insbesondere in Formulierungen mit anionischen Tensiden den Nachteil, daß die haaravivierenden und haarfestigenden Eigenschaften im Laufe längerer Lagerzeit allmählich verloren gehen.

Es ist weiterhin bekannt, nach dem Waschen oder Shampoonieren des Haares konditionierende Präparate, meist auf Basis kationischer Tenside, einwirken zu lassen oder den Haarwaschmitteln konditionierende Stoffe zuzusetzen, um gleichzeitig mit der Haarwäsche einen gewissen konditionierenden Effekt zu erzielen. Solche Substanzen sind zum Beispiel kationische Polymere wie kationische Cellulosederivate. So beschreibt die europäische Patentanmeldung EP 337 354 (Kao) Mittel, die eine Kombination von Alkylolykosiden und mindestens einem kationischen Polymer enthalten. Mit dieser Kombination sollen gemäß Aufgabenstellung der EP 337 354 Mittel erhalten werden, die hautschonend sind und ein gewisses Schaumvermögen aufweisen.

Die GB-A-2255101 beschreibt Shampoos, welche ein anionisches Tensid, ein Alkylglycosid und ein kationisches Polymer enthält.

Die deutsche Patentschrift DE 33 36 760 C2 (L'Oreal) beschreibt milde Reinigungs-Zusammensetzungen enthaltend Glucosid-Alkylether mit Alkylresten der Ketten länge $C_{8-10}$ und nichtionische Polymere wie Polyvinylpyrrolidon bzw. desssen Copolymeren mit nichtionischen Co-Monomeren. Die DE 33 36 760 geht dabei von der Aufgabenstellung aus, milde Reinigungsmittel zur Verfügung zu stellen.

Aus der europäischen Patentanmeldung EP 070 074 (Procter & Gamble) sind schäumende Tensidzusammensetzungen bekannt, die ein Alkylpolysaccharid-Tensid sowie ein anionisches Cotensid enthalten. In Beispiel XII ist darüber hinaus eine Shampoo-Zusammensensetzung offenbart, die ein alpha-Olefinsulfonat (anionisches Tensid), ein Alkylpolyglucosid und Hydroxyethylcellulose (ein halb-synthetisches nichtionisches Polymer) enthält. Das genannte Beispiel der EP 070 074 enthält jedoch keinen Hinweis darauf, wie die Wirk-Eigenschaften des Shampoos durch die Wahl von Art und Menge seiner Komponenten sich gezielt beeinflussen lassen; insbesondere enthält sie keinen Hinweis auf die vorteilhafte Wirkung rein synthetischer nichtionischer Polymere in entsprechenden ternären Mischungen.

Aus der deutschen Offenlegungsschrift DE-OS 27 32 734 (Colgate-Palmolive) sind Haarwaschmittel mit einem Gehalt an Aminoxiden, einem Fettsäureester, einem Alkylglycosid und einem Polyacrylamid bekannt. Gemäß der Beschreibung dieser Anmeldung kann als weiterer Hilfsstoff u.a. auch Polyvinylpyrrolidon vorhanden sein. Das genannte Mittel muß jedoch einerseits rein nichtionisch sein, d.h. es darf keinerlei Komponenten mit ionischem Charakter, wie etwa anionische Tenside, enthalten; andererseits bezieht sich die Lehre der DE-OS 27 32 734 auf eine ganz spezielle Kombination von vier speziellen Komponenten und enthält dementsprechend keinerlei Hinweis auf die vorteilhafte Wirkung ternärer Mischungen ganz anderer Natur wie sie in der hier vorliegenden Erfindung angesprochen sind.

**Beschreibung der Erfindung**

Das Anforderungsprofil, das an oberflächenaktive Mischungen der erfindungsgemäß betroffenen Art gestellt wird,

2

EP 0 681 468 B1

beschränkt sich jedoch nicht auf die geforderten hautschonenden Eigenschaften sowie ein gewisses Schaumvermögen, es sind vielmehr eine Vielzahl weiterer Forderungen zu erfüllen.

Mit den aus dem Stand der Technik - z.B. der EP 337 354 - bekannten Mitteln wird zwar eine befriedigende Verbesserung der Naßkämmbarkeit und auch eine Verringerung der statischen Aufladung erzielt. Diese Effekte gehen jedoch praktisch immer mit einer zu starken Glättung des trockenen Haares einher. Dadurch entsteht der Nachteil, daß das Haar wenig Fülle und die Frisur keinen Halt aufweist. Die Glätte des Haares ist um so ausgeprägter, je niedriger der Kämmwiderstand des trockenen Haares ist.

Es bestand daher die Aufgabe, oberflächenaktive Mischungen und insbesondere Mittel zum Waschen und Spülen von Haaren, aufzufinden, welche bei gleichzeitig gutem Schaumvermögen und geringer Hautbelastung die Frisierbarkeit und den Frisurenhalt des Haares verbessern, ohne eine Klebrigkeit des Haares zu verursachen.

Es wurde nun überraschend gefunden, daß Fülle, Frisierbarkeit und Frisurenhalt der Haare im Vergleich zum bekannten Stand der Technik deutlich verbessert werden, wenn die oberflächenaktive Mischung 1 bis 50 Gew.-% eines Aniontensids, 0,5 bis 15 Gew.-% eines Alkylglykosids und 0,1 bis 7 Gew.-% eines synthetischen nichtionischen Polymers enthält.

Gegenstand der Erfindung sind dementsprechend oberflächenaktive Mischungen enthaltend

(A) 1 bis 50 Gew.-% eines oder mehrerer Aniontenside, die 1 oder 2 lipophile Reste mit 1 bis 22 C-Atomen und einen polaren Rest ausgewählt aus der Gruppe der Carboxylat-, Sulfat- oder Sulfonatreste und gegebenenfalls einen Polyoxyalkylenrest mit einem mittleren Alkoxylierungsgrad von 1 bis 15 enthalten,

(B) 0,5 bis 15 Gew.-% eines oder mehrerer Alkylglycoside der allgemeinen Formel (I)

$$R(G)_x \hspace{6cm} (I)$$

worin R einen linearen, gesättigten Alkylrest mit 12 bis 22 C-Atomen und $(G)_x$ ein Glycosid- oder Oligoglycosidrest mit einem Oligomerisationsgrad x von 1 bis 10 ist,

(C) 0,1 bis 7 Gew.-% eines synthetischen nichtionischen Polymers, ausgewählt aus der Gruppe der Polyvinylpyrrolidone oder der Copolymeren von Vinylpyrrolidon mit Vinylacetat, Vinylpropionat, Vinylalkohol, Acryl- oder Methacrylsäureestern,

(D) 28 bis 98,4 Gew.-% Wasser,

mit der Maßgabe, daß der Gehalt der Mischungen an der Summe der Komponenten (B) und (C) nicht größer als der Gehalt an Komponente (A) ist.

Unter Polyoxyalkylenrest wird dabei eine Gruppe verstanden, die aus Oxyethylen-Einheiten $-[CH_2\text{-}CH_2\text{-}O]-$ oder aus Oxypropylen-Einheiten $-[CH(CH_3)\text{-}CH_2\text{-}O]-$ aufgebaut ist. Wie in der Fachwelt allgemein üblich wird dabei unter dem mittleren Alkoxylierungsgrad die mittlere Anzahl der Oxyalkylen-Einheiten pro Molekül des Aniontensids verstanden.

Die **Aniontenside (A)** werden erfindungsgemäß bevorzugt ausgewählt aus der Gruppe der Alkyl- und Dialkylethersulfate, Ethercarbonsäuren, Sulfobernsteinsäurehalbester, Fettalkoholethercitrate, Fettalkoholethertartrate, Acylsarkoside Acyltauride, Acylisethionate und der Sulfonate von ungesättigten Fettsäuren.

Die Gegenionen der Carboxylat- Sulfat oder Sulfonatreste werden bevorzugt aus der Gruppe der Alkali- und Erdalkalimetalle, Aluminium, Ammonium sowie Alkyl- oder Alkylolammoniumgruppen mit 1 bis 4 C-Atomen in jeder Alkyl- oder Alkylolgruppe gewählt. Ganz besonders geeignet ist die Gruppe der Alkalimetalle.

Die chemischen Strukturen sowie die grundlegenden Tensideigenschaften der meisten dieser Aniontenside gehören mittlerweile zum Lehrbuchwissen und bedürfen daher keiner weiteren Erläuterung. Weiter geeignete Aniontenside seien im folgenden genannt: Unter Dialkylethersulfaten sind Verbindungen zu verstehen wie sie in der Europäischen Patentameldung EP 299 370 beschrieben sind. Aus dieser Schrift können Einzelheiten des Herstellungsverfahrens und der Eigenschaften dieser Verbindungen entnommen werden. Unter Fettalkoholethertartraten sind Monoestersalze der Weinsäure, unter Fettalkoholethercitraten Mono-und/oder Diestersalze der Zitronensäure mit Anlagerungsprodukten von Ethylenoxid und/oder Propylenoxid an Fettalkohole zu verstehen. Unter Sulfonaten von ungesättigten Fettsäuren sind Sulfonierungsprodukte von Fettsäuren mit 12 bis 22 C-Atomen und 1 bis 6 Doppelbindungen zu verstehen. Derartige Produkte sind literaturbekannt und beispielsweise durch Umsetzung dieser Fettsäuren mit gasförmigem Schwefeltrioxid zugänglich. Am Beispiel der Ölsäure können Einzelheiten des Herstellungsverfahrens der deutschen Patentanmeldung DE 39 26 344 entnommen werden.

Bei Aniontensiden, die einen Polyoxyalkylenrest enthalten, gilt bezüglich des Alkoxylierungsgrades ganz allgemein, daß bei Alkoxylierungsreaktionen wie beispielsweise der Anlagerung von x mol Ethylenoxid an 1 mol Fettalkohol nach den bekannten Ethoxylierungsverfahren kein einheitliches Adduct, sondern ein Gemisch aus Restmengen freien Fettalkohols und einer Reihe homologer (oligomerer) Anlagerungsprodukte von 1, 2, 3, ... x, x+1, x+2 ... usw. Molekülen Ethylenoxid je Molekül Fettalkohol erhalten wird. Der mittlere Ethoxylierungsgrad (x) wird dabei definiert durch die Ausgangsmengen an Fettalkohol und Ethylenoxid. Die Verteilungskurve des Homologengemisches weist in der Regel ein Maximum im Bereich zwischen x-3 und x+3 auf. Nähere Informationen hierzu können beispielsweise der Zeitschrift Soap/Cosmetics/Chemical Specialities, Heft Januar 1988, S. 34, entnommen werden. Neben den üblichen aus dem Stand der Technik bekannten Alkoxylierungskatalysatoren wie Natriummethanolat lassen sich dabei aber auch solche Katalysatoren verwenden, die zu Produkten mit sogenannter eingeengter Homologenverteilung führen, vergl. z.B. Seifen-Öle-Fette-Wachse 1990 (116) 60.

In einer bevorzugten Ausführungsform der Erfindung beträgt der Anteil der anionischen Tenside 4 bis 30 Gew.-%. **Alkylglycoside (B)** der allgemeinen Formel (I)

$$R\text{-}(G)_x \tag{I}$$

worin R, G und x die oben genannte Bedeutung haben, sind seit langem bekannte oberflächenaktive Stoffe, die aus Zuckern und aliphatischen, primären Alkoholen unter Acetalisierung herstellbar sind. Als Zuckerkomponenten (Glycosen) kommen bevorzugt Glucose, daneben aber auch Fructose, Mannose, Galactose, Telose, Gulose, Allose, Altrose, Idose, Arabinose, Xylose, Lyxose, Libose und Gemische davon in Frage.

Bevorzugt wegen der leichten Zugänglichkeit und der guten Anwendungseigenschaften sind die Acetalisierungsprodukte der Glucose mit Fettalkoholen R-OH, die z. B. aus natürlichen Fetten und Ölen nach bekannten Verfahren erhältlich sind, insbesondere mit linearen, primären, gesättigten und ungesättigten Fettalkoholen mit 12 bis 22 C-Atomen.

Alkylglycoside, ihre Herstellung und Verwendung als oberflächenaktive Stoffe sind beispielsweise aus US-A-3.839.318, US-A-3.707.535, US-A-3.547.828, DE-A-1943689, DE-A-2036472, DE-A-3001064 sowie EP-A-77167 bekannt. Bezüglich des Glycosidrestes - $(G)_x$ gilt, daß sowohl Monoglycoside (x = 1), bei denen ein Zuckerrest glycosidisch mit dem Fettalkohol verbunden ist, als auch oligomere Glycoside mit einem Oligomerisationsgrad x = 2 bis 10 geeignet sind. In der Regel liegen Gemische von Mono- und Oligoglycosiden vor.

Ganz besonders geeignet sind im Sinne der Lehre der vorliegenden Erfindung solche Alkylglycoside (B), in welchen R eine Alkylgruppe mit 12 bis 16 C-Atomen und $(G)_x$ ein Glycosid- oder Oligoglycosidrest mit einem Oligomerisationsgrad x = 1 bis 10 ist. Ganz besonders bevorzugt sind dabei Gemische von Glucosid und Oligoglucosiden mit einem mittleren Oligomerisationsgrad von 1 bis 1,5.

In eine bevorzugten Ausführungsform der Erfindung beträgt der Anteil des Alkylglykosids 1 bis 8 Gew.-%.

Gemäß der Lehre der vorliegenden Erfindung werden als **Komponente (C)** ausdrücklich **synthetische nichtionische Polymere** eingesetzt. Unter der Bezeichnung "synthetisch" ist dabei zu verstehen, daß es sich um <u>rein</u> synthetische Produkte handelt. Produkte rein natürlichen Ursprungs oder halb-synthetisch Produkte, wie Hydroxyethylcellulose, Methyl-hydroxypropylcellulose (z.B. "Methocel 65 HG") und dergleichen fallen demgemäß <u>nicht</u> unter diese Definition.

Die Wahl dieser synthetischen nichtionischen Polymeren (C) unterliegt an sich keinen besonderen Einschränkungen. Als besonders gut geeignet haben sich im Sinne der erfindungsgemäßen Lehre jedoch diejenigen Polymeren (C) erwiesen, die Stickstoff-haltig sind. In diesem Zusammenhang sei nochmals ausdrücklich klargestellt, daß der Begriff "nichtionisch" nur solche Polymeren (C) umfaßt, die weder permanenten noch temporären kationischen Charakter aufweisen.

Unter Polymeren mit permanentem kationischen Charakter sind dabei solche Polymere zu verstehen, deren molekulare Struktur einerseits einen polymeren Teil mit permanenten kationischen Zentren aufweist, andererseits entsprechende Gegenionen anionischen Charakters. Unter "permanent" wird dabei verstanden, daß die positive Ladungen an den Heteroatomen wie Stickstoff zeitlich unveränderlich fixiert ist. Als typisches Beispiel seien die weithin bekannten quaternären Ammoniumsalze angeführt, bei denen der Stickstoff mit vier organischen Resten verknüpft ist. Die positive Ladung des Stickstoffs hat daher permanenten Charakter und bleibt solange erhalten, als die Verbindung nicht chemisch modifiziert, d.h. der Substitutionsgrad des Stickstoffs durch chemische Reaktion verändert wird.

In Anlehnung an den Begriff eines "permanenten" kationischen Polymeren ist unter einer "temporär" kationischen Form eines stickstoffhaltigen Polymeren zu verstehen, daß sich das Ausmaß der positiven Ladung der Stickstoffatome in einfacher Weise, etwa durch Einstellung des pH-Wertes der wäßrigen Mischung, modifizieren läßt. Strukturell wird dies dadurch ermöglicht, daß die Stickstoffatome der entsprechenden Polymeren nur mit drei organischen Resten verknüpft sind. Durch Einstellung eines sauren pH-Wertes wird dann der Stickstoff positiv geladen; diese Ladung läßt sich jedoch durch Änderung des pH-Wertes in Richtung eines alkalischen Milieus wieder entfernen, ohne daß die Struktur des Polymers dabei verändert wird.

Als ganz besonders vorteilhaft haben sich im Rahmen der vorliegenden Erfindung diejenigen Stickstoff-haltigen Polymeren (C) erwiesen, die ausgewählt sind aus der Gruppe der

C1) - Polyvinylpyrrolidone,
C2) - der Copolymeren von Vinylpyrrolidon mit Vinylacetat, Vinylpropionat, Vinylalkohol sowie mit Acryl- oder Methacrylsäureestern.

Schließlich können auch solche Copolymeren eingesetzt werden, die unter Verwendung von mehr als zwei Monomer-Bausteinen hergestellt wurden (C3).

Beisiele für handelsübliche Polyvinylpyrrolidone vom Typ (C1) sind Luviskol K30 und K90, für Copolymere vom Typ (C2) Luviskol VA 37 und VA 64. Zum Typ (C3) zählt beispielsweise Luviskol VAP, das aus Vinylpyrrolidon, Vinylacetat und Vinylpropionat hergestellt wird.

In einer bevorzugten Ausführungsform der Erfindung beträgt der Anteil des nichtionischen Polymers 0,5 bis 5 Gew.-%.

Es sei darauf hingewiesen, daß die angesprochenen Vorteile, die durch die erfindungsgemäßen Mischungen erzielt werden, auch ohne die Anwesenheit zusätzlicher Hilfsstoffe erzielt werden. Für die Formulierung in reinigenden und/oder pflegenden Produkten kann man jedoch weitere Komponenten verwenden, die - wie unten beschrieben - allgemein üblich sind.

Weiterhin können die erfindungsgemäßen Mittel neben den Aniontensiden (A) zusätzlich 0,5-20 Gew.-%, insbesondere 1-10 Gew.-%, an ampholytischen und/oder zwitterionischen Tensiden enthalten.

Unter **ampholytischen Tensiden** werden solche oberflächenaktiven Verbindungen verstanden, die außer einer $C_8$-$C_{18}$-Alkyl- oder -Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarkosine, 2-Alkylaminopropionsäuren und Alkylaminoessigsäuren mit jeweils etwa 8 bis 18 C-Atomen in der Alkylgruppe.

Als **zwitterionische Tenside** werden solche oberflächenaktiven Verbindungen bezeichnet, die im Molekül mindestens eine quartäre Ammoniumgruppe und mindestens eine -COO$^{(-)}$ oder -$SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinat, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethyl carboxymethylglycinat.

Die hautschonenden Eigenschaften der erfindungsgemäßen Mittel kommen besonders dann zur Geltung, wenn diese so formuliert werden, daß sie einen pH-Wert in der Nähe des Neutralpunktes der Haut aufweisen. Mittel mit pH-Werten im Bereich von 5,0 - 6,5 sind dabei bevorzugt.

Die erfindungsgemäßen Mittel können darüber hinaus **anorganische Elektrolytsalze (E)** enthalten. Dazu eignen sich alle wasserlöslichen Alkali-, Ammonium- und Erdalkalisalze, z. B. die Fluoride, Chloride, Bromide, Sulfate, Phosphate und Nitrate und Hydrogencarbonate, soweit sie in einer Menge von wenigstens 1 Gew.-% bei 20°C in Wasser löslich sind. Bevorzugt werden die Chloride oder Sulfate eines Alkalimetalls, des Ammoniums oder des Magnesiums verwendet; besonders bevorzugt sind Natriumchlorid und Magnesiumchlorid. Bevorzugt beträgt die Menge des Elektrolytsalzes 0,1 bis 10 Gew.-%.

Die erfindungsgemäßen Mittel können in einer Vielzahl von reinigenden und/oder pflegenden Konsumentenprodukten Verwendung finden. Dazu zählen beispielsweise kosmetische Mittel wie Haarshampoos, Schaumbädern, Duschbädern, flüssigen Seifen, abspülbare Haarnachbehandlungsmittel wie Haarspülungen, Haarkuren und dergleichen. Insbesondere eignen sie sich für milde Haarshampoos mit verbesserter Fülle und Frisierbarkeit der Haare. Die erfindungsgemäßen Mittel können aber auch in manuellen Geschirrspülmitteln Verwendung finden.

Diese Produkte enthalten neben Tensiden oder Tensidkombinationen üblicherweise Bestandteile wie Emulgatoren, Ölkomponenten, Lösungsvermittler, Verdickungsmittel, Überfettungsmittel, biogene Wirkstoffe, Filmbildner, Duftstoffe, Farbstoffe, Perlglanzmittel, Schaumstabilisatoren, konservierungsmittel und pH-Regulatoren. Die erfindungsgemäßen Mittel können daher zusätzliche Komponenten und Hilfsstoffe enthalten, wie sie aus dem Stand der Technik bekannt sind. Die wichtigsten sind:

**Nichtionische Tenside/Emulgatoren**, z.B. Ethylenoxidaddukte an Alkohole, Carbonsäuren, Partialglyceride und Sorbitanester sowie z.B. Fettsäureester und Sorbitanfettsäureester (vergl. z.B. W. Umbach [Hrsg.], "Kosmetik - Entwicklung, Herstellung und Anwendung kosmetischer Mittel", S.86-87, Stuttgart 1988).

**Ölkomponenten**, z.B. Substanzen wie Paraffinöl, Pflanzenöle, Fettsäureester, Squalan und 2-Octyldodecanol; als Fette und Wachse beispielsweise Walrat, Bienenwachs, Montanwachs, Paraffin und Cetyl-stearylalkohol.

**Lösungsvermittler**, z.B. niedrige ein- oder mehrwertige Alkohole wie Ethanol, Isopropanol, Ethylenglykol, Propylenglykol, Glycerin, 1,3-Butylenglykol und Diethylenglykol.

**Verdickungsmittel,** z.B. Polysaccharide, insbesondere Xanthan-Gum, Guar-Gum, Agar-Agar, Alginate und Tylosen, sowie Carboxymethylcellulose und Hydroxyethylcellulose, ferner höhermolekulare Polyethylenglykolmono- und -diester von Fettsäuren, Polyacrylate, Polyvinylalkohol.

**Überfettungsmittel**, z.B. polyethoxylierte Lanolinderivate, Lecithinderivate und Fettsäurealkanolamide, wobei die letzteren gleichzeitig auch als Schaumstabilisatoren dienen.

**Biogenen Wirkstoffe** wie Pflanzenextrakte, Eiweißabbauprodukte und Vitaminkomplexe.

**Feuchthaltemittel,** z.B. Glycerin, Polyglycerine, Sorbit, 1,2-Propandiol, 1,2,3-Butan-triol, Polyethylenglykole, Glucose, Mannit, Xylit, Pyrollidon-Carbonsäure-Salze (PCA), Aminosäuren, Milchsäure.

**Antimikrobiell wirksame Stoffe** als Konservierungsmittel, z.B. Benzoesäure, Salicylsäure, Sorbinsäure, sowie deren Ester und Salze sowie die in der Anlage zur Kosmetikverordnung aufgeführten Substanzen.

**Perlglanzmittel** wie Glykoldistearinsäureester, Ethylenglykoldistearat oder Fettsäuremonoglykolester.

**Duftstoffe,** z.B. natürliche Riechstoffe, die durch Destillation, Extraktion oder Pressung aus Pflanzen gewonnen werden sowie synthetisch hergestellte Riechstoffe (vergl. z.B. H.Aebi, E.Baumgartner, H.P.Fiedler, G.Ohloff, "Kosmetika, Riechstoffe und Lebensmittelzusatzstoffe", Stuttgart 1978).

**Antioxidantien,** z.B. Tocopherole, Lecithin, Guajakol, Butylkresol, 4-Methyl-2,6-di-tert.-butyl-phenol (BHT), 4-Methoxy-2(3)tert.-butylphenol (BHA)

**Farbstoffe,** wie sie z.B. von der Farbstoff-Kommission der Deutschen Forschungsgemeinschaft für Kosmetika zusammengestellt sind ("Färbemittel für Kosmetika" Mitteilung 3, Wiesbaden 1968). Die Farbstoffe werden üblicherweise in Konzentrationen von 0,001 bis 0,1 Gew.-%, bezogen auf die gesamte Mischung, eingesetzt.

**pH-Regulatoren:** Weitere Komponenten der erfindungsgemäßen Mittel sind gewünschtenfalls Substanzen, die der Einstellung des pH-Wertes der Mittel dienen.

Die Gesamtmenge der Hilfsstoffe beträgt 0 - 20 Gew.-%, vorzugsweise 0 - 10 Gew.-%.

Zur Herstellung der erfindungsgemäßen Mischungen wird das Alkylglycosid (B) bei 50 bis 80 °C zu einer wäßrigen Phase gegeben, die die Aniontenside (A) und das synthetische nichtionische Polymer (C) enthält. Diese Mischungen werden gerührt und dann auf Normaltemperatur (20 bis 40 °C) abgekühlt. Wegen der Wasserlöslichkeit der Alkylglycoside, insbesondere solcher mit Alkylresten von 12 bis 16 C-Atomen, können die erfindungsgemäßen Mittel vorteilhaft auch auf kaltem Wege hergestellt werden. Dabei werden die Komponenten in einfacher Weise bei Normaltemperatur zusammengerührt.

Die nachfolgenden Beispiele dienen der Erläuterung der Erfindung und sind nicht einschränkend aufzufassen.

## Beispiele

### 1. Allgemeines

#### 1.1. Abkürzungen

In den Kopfzeilen der Tabellen 1 bis 5 sind die Beispiele mit **B1** bis **B5**, die Vergleiche mit **V1** bis **V10** kenntlich gemacht.

#### 1.2. Verwendete Substanzen

##### 1.2.1. Tenside

**N25:** Wäßrige Lösung von Natriumlaurylethersulfat; Aktivsubstanzgehalt: 28 Gew.-% ("Texapon[(R)] N25"; Fa. Henkel/Düsseldorf)

**Dehyton K:** Wäßrige Lösung eines Fettsäureamid-Derivats mit Betainstruktur der Formel R-CONH-$(CH_2)_3$-$N^+(CH_3)_2$-$CH_2$-COO- ; CTFA-Bezeichnung: Cocamidopropyl Betaine; Aktivsubstanzgehalt: 30 Gew.-%; NaCl-Gehalt: 5 Gew.-% (Fa. Henkel/Düsseldorf)

**AKYP:** Wäßrige Lösung eines Ethercarbonsäuresalzes der Formel $C_{12/14}$-$(O$-$CH_2$-$CH_2)_{10}$-$OCH_2$-COONa, Aktivsubstanzgehalt: 22 Gew. % ("Akypo[(R)]-Soft 100 NV"; Fa. Chemy-Y)

**APG600:** $C_{12/14}$-Fettalkoholglucosid mit einem Oligomerisationsgrad von 1,45 (Fa. Henkel/Düsseldorf).

**Texapon SB3:** Wäßrige Lösung eines Sulfobernsteinsäurehalbesters auf Basis eines Alkylpolyglykolethers, Di-Na-Salz, CTFA-Bezeichnung: Disodiumlaureth, (C12, 12 (3 EO)) Sulfosuccinate; Aktivsubstanzgehalt: 40 Gew.-% ("Texapon[(R)] SB3"; Fa. Henkel/Düsseldorf)

**Texapon ASV:** Wäßrige Lösung spezieller Alkyl- und Alkylethersulfate. CTFA-Bezeichnung: sodium laureth sulfate and magnesium laureth sulfate and sodium laureth-8-sulfate and magnesium laureth-8-sulfate and sodium oleate sulfate and magnesium oleate sulfate. AS-Gehalt: 28 Gew.-% ("Texapon[(R)] ASV"; Fa. Henkel/Düsseldorf)

**Dehyton CB:** Fettaminderivat mit Betainstruktur; CTFA-Bezeichnung: Coco-Betain; Aktivsubstanzgehalt: 31 Gew.-%; NaCl-Gehalt 5 Gew.-% (Fa. Henkel /Düsseldorf)

**Dehyton AB30:** Wäßrige Lösung eines Dimethylkokosalkylammoniumbetains; Aktivsubstanzgehalt: 30 Gew.-% ("Dehyton[(R)] AB30"; Fa. Henkel/Düsseldorf)

**Texapon K14S:** Wäßrige Lösung von Natriumlaurylmyristylethersulfat; Aktivsubstanzgehalt: 28 Gew.-% ("Texapon[(R)] K14S"; Fa. Henkel/Düsseldorf)

**Genamin DSAC:** Dimethyldistearylammoniumchlorid ("Genamin-DS-AC"; Fa. Hoechst)

**Dehyquart A:** Wäßrige Lösung von Trimethylhexadecylammoniumchlorid; CTFA-Bezeichnung: Cetrimonium Chloride; Aktivsubstanzgehalt: 25 Gew.-% ("Dehyquart$^{(R)}$ A"; Fa. Henkel/Düsseldorf)

### 1.2.2. Synthetische nichtionische Polymere

**PVP:** Polyvinylpyrrolidon ("Luviskol K30"; Fa. BASF)
**COPOLY-1:** Vinylpyrrolidon/Vinylacetat-Copolymer ("Luviskol VA64"; Fa. BASF)
**COPOLY-2:** Vinylpyrrolidon/Vinylacetat/Vinylpropionat-Copolymer ("Luviskol VAP 343 E"; Fa. BASF)

### 1.2.3. Sonstige Stoffe

**Nutrilan I:** Eiweiß-Hydrolysat; CTFA-Bezeichnung: hydrolyzed-animal-collagen (Nutrilan$^{(R)}$ I"; Fa. Grünau/Illertissen)
**Eucarol TA:** ($C_{12/14}$-Alkohol + 7 EO) monotartrat; Weinsäureester eines Anlagerungsproduktes von 7 Mol Ethylenoxid an einen $C_{12/14}$-Fettalkohol ("Eucarol TA"; Fa. Auschem/Mailand)
**Lanette O:** Cetyl-/Stearylalkohol [50:50] (Fa. Henkel/Düsseldorf)
**Polymer W 37194:** Acrylamidopropyltrimethylammoniumchlorid/Acrylsäure-Copolymer. d.h. ein zwitterionisches Polymer (Fa. Stockhausen)

### 2. Bestimmung der Trockenkämmbarkeit

Den Untersuchungen zur Kämmbarkeit wurde die Methode gemäß J. Soc. Cosm. Chem. 1973 [24] 782 zugrundegelegt.

Die Trockenkämmarbeit wurde an braunem Haar (Alkinco #6634, Strähnenlänge 12 cm, Strähnenmasse 1 g) untersucht. Es handelte sich um leicht vorgeschädigte (blondierte) Haare, wie sie etwa beim durchschnittlichen Anwender zu erwarten sind. Nach der Nullmessung wurden die Strähnen mit 100 ml der zu prüfenden Rezeptur getränkt. Nach 5 Minuten Einwirkzeit wurden die Strähnen 1 Minute unter fließendem Wasser (1 l/min, 38 °C) ausgespült. Die Strähnen wurden anschließend 12 Stunden bei 30 °C und einer relativen Luftfeuchtigkeit von 20% getrockhet und dann erneut vermessen und das Ergebnis mit der Nulimessung verglichen. Die Ergebnisse der Trockenkämmbarkeitsversuche sind in der Tabelle 1 zusammengestellt.

Tabelle 1

| Trockenkämmbarkeit[1] | | | |
|---|---|---|---|
| Substanz | V1 | V2 | B1 |
| N25 | 9,0 | 9,0 | 9,0 |
| AKYP | 3,0 | 3,0 | 3,0 |
| Dehyton K | 3,0 | 3,0 | 3,0 |
| Nutrilan I | 0,5 | 0,5 | 0,5 |
| PVP | - | 1,0 | 1,0 |
| APG 600 | - | - | 2,0 |
| Wasser | ad 100 | ad 100 | ad 100 |
| TK (%)[2] | 95 | 110 | 135 |

[1] Die Angaben im oberen Block der Tabelle beziehen sich auf Gew.-% Aktivsubstanz. Die Angaben im unteren Block der Tabelle beziehen sich auf den experimentell ermittelten Wert der Trockenkämmbarkeit TK im Vergleich zur Nullmessung.
[2] Die statistische Sicherheit der TK-Werte betrug 99,99%.

Das erfindungsgemäße Beispiel B1 weist eine im Vergleich zu V1 und V2 deutlich höhere Trockenkämmbarkeit auf. Da TK ein Maß für den Halt der Frisur ist, ist für B1 eine Verbesserung des Frisurenhalts nachgewiesen.

### 3. Bestimmung des Lockenhaltevermögens

Eine 15 cm lange Haarsträhne (Alkinco 6634; Strähnengewicht: 2 g) wurde auf ein Glasrohr mit einem Außendurchmesser von 1,7 cm gewickelt, fixiert und mit 0,2 g der zu prüfenden Rezeptur behandelt. Anschließend wurde die Haarsträhne mit Wasser gespült und getrocknet. Ein Maß für die Stabilität der nach dem Herausziehen des Glasstabes erhaltenen Locke ist der Curl-Retention-Wert. Der Curl-Retention-Wert (CR-Wert) ist definiert als $[(l-l_x)/(l-l_o)] * 100 \%$, wobei 1 die Länge der Haarsträhne (15 cm), $l_o$ die Länge der Haarlocke unmittelbar nach dem Trocknen und $l_x$ die Länge der Haarlocke nach 48-stündiger Lagerung in einem Trockenschrank bei konstanten Bedingungen (30 °C, 40 % relative Luftfeuchtigkeit) ist. Die Ergebnisse der Messungen sind in Tabelle 3 zusammengestellt.

Tabelle 2

| Lockenhaltevermögen[1] | | | |
|---|---|---|---|
| Substanz | V3 | V4 | B2 |
| N25 | 9,0 | 9,0 | 9,0 |
| AKYP | 3,0 | 3,0 | 3,0 |
| Dehyton K | 3,0 | 3,0 | 3,0 |
| Nutrilan I | 0,5 | 0,5 | 0,5 |
| PVP | - | 1,0 | 1,0 |
| APG 600 | - | - | 2,0 |
| Wasser | ad 100 | ad 100 | ad 100 |
| CR-Wert (%) | 84,5 | 94,3 | 95,8 |

[1] Die Angaben im oberen Block der Tabelle beziehen sich auf Gew.-% Aktivsubstanz. Die Angaben im unteren Block der Tabelle beziehen sich auf den experimentell ermittelten CR-Wert.

### 4. Elektrostatische Aufladung

Zur Bestimmung der elektrostatischen Aufladung beim Kämmen wurde eine Haarsträhne innerhalb eines doppelten Faraday-Käfigs aufgehängt. Zwischen innerem und äußerem Käfig bestand keine leitende Verbindung. Der äußere Käfig war mit dem Nulipotential (Erde) verbunden. Ein Voltmeter maß Potentialdifferenzen zwischen innerem und äußerem Käfig. Potentialdifferenzen entstehen immer dann, wenn aufgrund von Reibungsarbeiten beim Kämmen Ladungen von den Oberflächen der Haarfasern abgezogen werden und sich auf der Kammoberfläche sammeln. Das elektrische Feld und die im Vergleich zum Kamm entgegengesetzt aufgeladene Strähne tritt mit dem inneren Faraday-Käfig in Wechselwirkung. Dieser lädt sich auf der Innenseite entgegengesetzt auf (Influenz). Die Ladungsverschiebung gegenüber dem äußeren Käfig wurde am Voltmeter registriert und war ein direktes Maß für die triboelektrische Aufladung der gekämmten Haarsträhne.

Tabelle 3

| Elektrostatische Aufladung[1] | | | |
|---|---|---|---|
| Substanz | V5 | V6 | B3 |
| N25 | 9,0 | 9,0 | 9,0 |
| AKYP | 3,0 | 3,0 | 3,0 |
| Dehyton K | 3,0 | 3,0 | 3,0 |
| Nutrilan I | 0,5 | 0,5 | 0,5 |
| PVP | - | 1,0 | 1,0 |
| APG 600 | - | - | 2,0 |
| Wasser | ad 100 | ad 100 | ad 100 |
| EA (%) | 92 | 87 | 75 |

[1] Die Angaben im oberen Block der Tabelle beziehen sich auf Gew.-% Aktivsubstanz. Die Angaben im unteren Block der Tabelle beziehen sich auf den experimentell ermittelten Wert der elektrostatischen Aufladung EA im Vergleich zu Nullmessung.

Das erfindungsgemäße Beispiel B3 zeigt einen im Vergleich zu V5 und V6 wesentlich geringeren Wert der elektrostatischen Aufladung. Geringere Werte für EA bedeuten eine verminderte Tendenz der Haare, sich gegenseitig abzustoßen und auseinanderzustreben ("fly-away-Effekt").

## 5. Bestimmung des Schaumverhaltens

Das Schaumverhalten der Reinigungsmittel wurde mit einer motorisierten Schlag-Schaum-Apparatur in Anlehnung an DIN 53902 bestimmt. Dazu wurden 340 ml einer Lösung hergestellt, die durch Verdünnen des wäßrigen Reinigungsmittels mit Leitungswasser aus Düsseldorf-Holthausen mit 18 ° dH derart hergestellt worden war, daß sie 2 Gew.-% Aktivsubstanz an Tensiden enthielt. Der Schaum wurde bei Raumtemperatur mit einer Lochplatte (Bohrungen von 1 mm Durchmesser, 10 Schläge bei einer Frequenz von 50 Schlägen/min, 13 cm Hub) erzeugt; er war sehr feinporig und entsprach somit weitgehend einem beim Shampoonieren auf dem Kopf entstehenden Schaum. Die Messungen wurden ohne Fettbelastung der Tensidlösung als Doppelbestimmung durchgeführt.

Als Standard wurde das Schaumverhalten einer als gut schäumend bekannten Mischung aus

(a) 42 Gewichtsprozent Texapon N 25 (= 12 % Aktivsubstanz),
(b) 10 Gewichtsprozent Dehyton K (= 3 % Aktivsubstanz),
(c) 1 Gewichtsprozent Comperlan LS (= 1 % Aktivsubstanz) und
(d) 47 Gewichtsprozent Wasser

bestimmt. Diese Standard-Mischung wurde mit Wasser verdünnt und ebenfalls in Form einer Lösung mit 2 Gew.-% Aktivsubstanz eingesetzt. Es wurden folgende Schaummengen gemessen:

- nach 1 Minute:      240 ml
- nach 3 Minuten:    210 ml
- nach 5 Minuten:    190 ml

Tabelle 4

| Relative Schaummenge | | | |
|---|---|---|---|
| Substanz | V7 | V8 | B4 |
| N25 | 9,0 | 9,0 | 9,0 |
| AKYP | 3,0 | 3,0 | 3,0 |
| Dehyton K | 3,0 | 3,0 | 3,0 |
| Nutrilan I | 0,5 | 0,5 | 0,5 |
| PVP | - | 1,0 | 1,0 |
| APG 600 | - | - | 2,0 |
| Wasser | ad 100 | ad 100 | ad 100 |
| Schaum 1' | 87 | 90 | 103 |
| Schaum 3' | 95 | 90 | 101 |
| Schaum 5' | 94 | 87 | 110 |

[1] Die Angaben im oberen Block der Tabelle beziehen sich auf Gew.-% Aktivsubstanz des wäßrigen Reinigungsmittels vor dem weiteren Verdünnen mit Wasser zu einer Lösung mit einem Gehalt an 2 % Aktivsubstanz an Tensiden. Die Angaben im unteren Block der Tabelle geben die prozentualle Schaummenge im Vergleich zum Standard an.

Das erfindungsgemäße Beispiel B4 zeigt eine im Vergleich zu V7 und V8 deutlich höhere Schaummenge.

## 6. Hautverträglichkeits-Untersuchungen

Zur Bestimmung der Hautverträglichkeit der Reinigungsmittel wurde die von Zeidler und Reese entwickelte in-vitro-Methode verwendet, die in der Zeitschrift Ärztliche Kosmetologie 13, 39-45 (1983) ausführlich dargestellt ist. Als Maß für die Hautverträglichkeit der Reinigungsmittel diente die Quellung von Schweine-Epidermis. Dazu wurde die benötigte Epidermis unmittelbar nach der Schlachtung junger Schweine gewonnen und tiefgekühlt gelagert.

Für die Messung wurden ausgestanzte Epidermisstreifen der Größe 1 cm x 6 cm 30 Minuten lang in eine Lösung getaucht, die durch Verdünnen des wäßrigen Reinigungsmittels mit Wasser derart hergestellt worden war, daß sie 2 Gew.-% Aktivsubstanz an Tensiden enthielt. Die Lösung war auf 39 °C temperiert und auf pH = 6,5 eingestellt. Sodann wurde nach kurzem Spülen und Entfernen des anhaftenden Wasser durch leichtes Abpressen unter definierten Bedingungen das Gewicht der gequollenen Streifen bestimmt. Anschließend wurden die Streifen 24 Stunden über Calciumchlorid entwässert und erneut gewogen. Um Einflüsse auszuschalten, die auf spezifische Eigenschaften des jeweiligen Tieres oder den Entnahmeort (Rücken, Seite) zurückgehen, wurde jeweils eine Standardmessung durchgeführt. Dabei wird ein unmittelbar benachbarter Epidermisstreifen in gleicher Weise mit Wasser anstelle des wäßrigen Reinigungsmittels behandelt.

Die Meßwerte t für die Tensid-Behandlung und w für die Behandlung mit Wasser ergeben sich aus der Beziehung:

$$t, w = \frac{\text{Gewicht(gequollene Epidermis) - Gewicht(trockene Epidermis)}}{\text{Gewicht(trockene Epidermis)}}$$

Die standardisierte, relative Quellungsänderung Q ist schließlich definiert als

$$Q = ( t/w - 1 ) * 100 \%$$

Der Q-Wert der wasserbehandelten Haut ist somit definitionsgemäß 0 %; negative Werte weisen auf quellungshemmende Eigenschaften hin.

Die Ergebnisse der Quellungsmessungen sind in der Tabelle 5 zusammengestellt. Es stellte sich heraus, daß das erfindungsgemäße Beispiel B5 im Vergleich zu V9 und V10 verbesserte Quellwerte aufweist.

Tabelle 5

| Quellungsmessungen[1] | | | |
|---|---|---|---|
| Substanz | V9 | V10 | B5 |
| N25 | 9,0 | 9,0 | 9,0 |
| AKYP | 3,0 | 3,0 | 3,0 |
| Dehyton K | 3,0 | 3,0 | 3,0 |
| Nutrilan I | 0,5 | 0,5 | 0,5 |
| PVP | - | 1,0 | 1,0 |
| APG 600 | - | - | 2,0 |
| Wasser | ad 100 | ad 100 | ad 100 |
| Quellwert[2] | 50 | 42 | 25 |

[1] Die Angaben im oberen Block der Tabelle beziehen sich auf Gew.-% Aktivsubstanz des wäßrigen Reinigungsmittels vor dem weiteren Verdünnen mit Wasser zu einer Lösung mit einem Gehalt an 2 % Aktivsubstanz an Tensiden. Die Angaben im unteren Block der Tabelle beziehen sich auf den experimentell ermittelten Quellwert Q.
[2] Die Fehlergrenzen betrugen bei allen Versuchen ± 7.

## 7. Anwendungsbeispiele

In den folgenden Beispielen sind exemplarisch typische Rezepturen auf Basis der erfindungsgemäßen oberflächenaktiven Mischungen angegebenen. Alle %-Angaben sind dabei als Gew.-% Aktivsubstanz zu verstehen. Die Differenz zu 100 % ist jeweils Wasser.

### 7.1. Shampoo

12,0 %    N 25
3,0 %     Dehyton K
2,0 %     APG 600
0,5 %     PVP
0,3 %     Polymer W 37194

### 7.2. Shampoo

6,0 %     K 14S
3,0 %     AKYP
6,0 %     Eucarol TA
3,0 %     Dehyton CB
3,0 %     APG 600
0,7 %     Polymer W 37194
1,5 %     COPOLY-1

### 7.3. Shampoo

3,0 %     Texapon SB3
10,0 %    Texapon ASV
2,5 %     Dehyton AB 30
5,0 %     APG 600

2,0 %   COPOLY-2

**7.4. Färbemittel**

4,0 %   Kokoslorol
4,0 %   Talgfettalkohol
4,0 %   N 25
1,0 %   APG 600
1,0 %   PVP
0,6 %   Polymer W 37194
0-4 %   Oxidationsfarbstoff
0-4 %   Direktziehender Haarfarbstoff

**Patentansprüche**

1.  Oberflächenaktive Mischungen enthaltend

    (A) 1 bis 50 Gew.-% eines oder mehrerer Aniontenside, die 1 oder 2 lipophile Reste mit 1 bis 22 C-Atomen und einen polaren Rest ausgewählt aus der Gruppe der Carboxylat-, Sulfat- oder Sulfonatreste und gegebenenfalls einen Polyoxyalkylenrest mit einem mittleren Alkoxylierungsgrad von 1 bis 15 enthalten,
    (B) 0,5 bis 15 Gew.-% eines oder mehrerer Alkylglycoside der allgemeinen Formel (1)

    $$R(G)_x \qquad\qquad (I)$$

    worin R einen linearen, gesättigten Alkylrest mit 12 bis 22 C-Atomen und $(G)_x$ ein Glycosid- oder Oligoglycosidrest mit einem Oligomerisationsgrad x von 1 bis 10 ist,
    (C) 0,1 bis 7 Gew.-% eines synthetischen nichtionischen Polymers, ausgewählt aus der Gruppe der Polyvinylpyrrolidone oder der Copolymeren von Vinylpyrrolidon mit Vinylacetat, Vinylpropionat, Vinylalkohol, Acryl- oder Methacrylsäureestern,
    (D) 28 bis 98,4 Gew.-% Wasser, mit der Maßgabe, daß der Gehalt der Mischungen an der Summe der Komponenten (B) und (C) nicht größer als der Gehalt an Komponente (A) ist.

2.  Oberflächenaktive Mischungen nach Anspruch 1, wobei das Alkylglykosid (B) ausgewählt ist aus der Gruppe derjenigen Verbindungen der Formel (I), bei denen R einen linearen, gesättigten Alkylrest mit 12 bis 16 C-Atomen bedeutet.

3.  Oberflächenaktive Mischungen nach einem der Ansprüche 1 bis 2, worin das synthetische nichtionische Polymer (C) ein Polyvinylpyrrolidon ist.

4.  Oberflächenaktive Mischungen nach einem der Ansprüche 1 bis 3, worin der Anteil der Aniontenside (A) 4 bis 30 Gew.-% beträgt.

5.  Oberflächenaktive Mischungen nach einem der Ansprüche 1 bis 4, worin der Anteil des Alkylglykosids (B) 1 bis 8 Gew.-% beträgt.

6.  Oberflächenaktive Mischungen nach einem der Ansprüche 1 bis 5, worin der Anteil des synthetischen nichtionischen Polymers (C) 0,5 bis 5 Gew.-% beträgt.

7.  Oberflächenaktive Mischungen nach einem der Ansprüche 1 bis 6, die zusätzlich 0,1 bis 10 Gew.-% eines anorganischen Elektrolytsalzes (E) enthalten.

8.  Verwendung der oberflächenaktiven Mischungen nach einem der Ansprüche 1 bis 7 in abspülbaren kosmetischen Formulierungen.

9.  Verwendung der oberflächenaktiven Mischungen nach einem der Ansprüche 1 bis 7 in reinigenden kosmetischen Produkten wie Haarshampoos, Schaumbädern, Duschbädern, flüssigen Seifen und dergleichen.

10. Verwendung der oberflächenaktiven Mischungen nach einem der Ansprüche 1 bis 7 in pflegenden kosmetischen Produkten wie Haarspülungen, Haarkuren und dergleichen.

**11.** Verwendung der oberflächenaktiven Mischungen nach einem der Ansprüche 1 bis 7 in Spül- und Reinigungsmitteln.

**Claims**

**1.** Surface-active mixtures containing

(A) 1 to 50% by weight of one or more anionic surfactants containing 1 or 2 lipophilic groups with 1 to 22 carbon atoms and a polar group selected from carboxylate, sulfate or sulfonate groups and, optionally, a polyoxyakylene group having an average degree of alkoxylation of 1 to 15,

(B) 0.5 to 15% by weight of one or more alkyl glycosides corresponding to general formula (I):

$$R(G)_x \qquad\qquad (I)$$

in which R is a linear saturated alkyl group containing 12 to 22 carbon atoms and $(G)_x$ is a glycoside or oligoglycoside unit with a degree of oligomerization x of 1 to 10,

(C) 0.1 to 7% by weight of a synthetic nonionic polymer selected from the group of polyvinyl pyrrolidones or copolymers of vinyl pyrrolidone with vinyl acetate, vinyl propionate, vinyl alcohol, acrylates or methacrylates,

(D) 28 to 98.4% by weight of water, with the proviso that the content of the mixtures in the sum of components (B) and (C) is no greater than the content of component (A).

**2.** Surface-active mixtures as claimed in claim 1, in which the alkyl glycoside (B) is selected from the group of those compounds corresponding to formula (I) where R is a linear saturated alkyl group containing 12 to 16 carbon atoms.

**3.** Surface-active mixtures as claimed in claims 1 and 2, in which the synthetic nonionic polymer (C) is a polyvinyl pyrrolidone.

**4.** Surface-active mixtures as claimed in any of claims 1 to 3, in which the content of anionic surfactants (A) is 4 to 30% by weight.

**5.** Surface-active mixtures as claimed in any of claims 1 to 4, in which the content of alkyl glycoside (B) is 1 to 8% by weight.

**6.** Surface-active mixtures as claimed in any of claims 1 to 5, in which the content of synthetic nonionic polymer (C) is 0.5 to 5% by weight.

**7.** Surface-active mixtures as claimed in any of claims 1 to 6 which additionally contain 0.1 to 10% by weight of an inorganic electrolyte salt (E).

**8.** The use of the surface-active mixtures claimed in any of claims 1 to 7 in cosmetic formulations removable by rinsing.

**9.** The use of the surface-active mixtures claimed in any of claims 1 to 7 in cosmetic cleaning products, such as hair shampoos, foam baths, shower baths, liquid soaps and the like.

**10.** The use of the surface-active mixtures claimed in any of claims 1 to 7 in cosmetic care products, such as hair rinses, hair conditioners and the like.

**11.** The use of the surface-active mixtures claimed in any of claims 1 to 7 in dishwashing detergents and cleaners.

**Revendications**

**1.** Mélanges tensioactifs renfermant

(A) 1 a 50 % en poids d'un ou de plusieurs surfactifs anioniques, qui possèdent 1 ou 2 radicaux lipophiles comportant 1 à 22 atomes de C et un radical polaire sélectionné parmi le groupe constitué des radicaux carboxylate, sulfate ou sulfonate et, le cas échéant, un radical polyoxyalkylène présentant un degré d'alcoxylation moyen compris entre 1 et 15,

(B) 0,5 à 15 % en poids d'un ou de plusieurs alkylglycosides de la formule générale (I)

$$R(G)_x \hspace{6cm} (I)$$

dans laquelle R représente un radical alkyle linéaire saturé comportant 12 à 22 atomes de C, et $(G)_x$ correspond à un radical glycoside ou oligoglycoside présentant un degré d'oligomérisation x de 1 à 10,
(C) 0,1 à 7 % en poids d'un polymère non ionique synthétique, sélectionné parmi le groupe constitué des polyvinylpyrrolidones ou des copolymères de vinylpyrrolidone et d'acétate vinylique, de propionate vinylique, d'alcool vinylique, d'acrylate ou de méthacrylate,
(D) 28 à 98,4 % en poids d'eau, à condition que la somme des concentrations des composants (B) et (C) dans les mélanges ne soit pas supérieure à la concentration en composant (A).

2. Mélanges tensioactifs selon la revendication 1, dans lesquels l'alkylglycoside (B) est sélectionné parmi le groupe constitué des composés de formule (I), dans lesquels R représente un radical alkyle linéaire, saturé, comportant 12 à 16 atomes de carbone.

3. Mélanges tensioactifs selon une des revendications 1 à 2, dans lesquels le polymère non ionique synthétique (C) est une polyvinylpyrrolidone.

4. Mélanges tensioactifs selon une des revendications 1 à 3, dans lesquels la proportion des surfactifs anioniques (A) atteint 4 à 30 % en poids.

5. Mélanges tensioactifs selon une des revendications 1 à 4, dans lesquels la proportion de l'alkylglycoside (B) atteint 1 à 8 % en poids.

6. Mélanges tensioactifs selon une des revendications 1 à 5, dans lesquels la proportion du polymère non ionique synthétique (C) atteint 0,5 à 5 % en poids.

7. Mélanges tensioactifs selon une des revendications 1 à 6, qui renferment en plus 0,1 à 10 % en poids d'un sel électrolytique inorganique (E).

8. Utilisation des mélanges tensioactifs selon une des revendications 1 à 7 dans des formulations cosmétiques rinçables.

9. Utilisation des mélanges tensioactifs selon une des revendications 1 à 7 dans des produits cosmétiques détergents, tels que shampooings pour cheveux, bains moussants, bains-douches, savons liquides et autres produits similaires.

10. Utilisation des mélanges tensioactifs selon une des revendications 1 à 7 dans des produits cosmétiques soignants, tels que rinçages, cures et autres produits capillaires similaires.

11. Utilisation des mélanges tensioactifs selon une des revendications 1 à 7 dans des produits de rinçage et de nettoyage.